# NEW EUROPEAN PATENT SPECIFICATION

(11) **EP 1 382 399 B2**
(45) Date of publication and mention of the opposition decision: **16.09.2015**
(45) Mention of the grant of the patent: 18.07.2007
(21) Application number: 03076766.9
(22) Date of filing: 12.08.1998
(51) Int. Cl.: B05B 17/06, A01M 1/20

(54) **Method for repelling or eliminating harmful organism**
Verfahren zur Vertreibung oder Beseitigung von Schädlingen
Procédé pour repousser ou éliminer les organismes nuisibles

(30) Priority: 20.08.1997 JP 22411797; 04.12.1997 JP 33440097; 09.03.1998 JP 5563698
(43) Date of publication of application: 21.01.2004
(62) Divisional of application: 98306421.3
(73) Proprietor: FUMAKILLA LIMITED, Tokyo (JP)
(72) Inventor: Abe, Toshio, Hiroshima-ken (JP); Yamazaki, Satoshi, Hiroshima-ken (JP); Sugiura, Masaaki, Hiroshima-ken (JP); Orita, Kunitaka, Hiroshima-ken (JP)
(74) Representative: Brearley, Helen Rebecca

(56) References cited:
- WO-A1-95/19304
- WO-A1-98/00010
- DE-A1- 1 625 230
- US-A- 3 643 836
- US-A- 4 173 651
- US-A- 4 173 651
- US-A1- 3 310 466
- PATENT ABSTRACTS OF JAPAN vol. 001, no. 045 (C-011), 4 May 1977 (1977-05-04) & JP 52 000660 A (FUMAKIRAA KK), 6 January 1977 (1977-01-06)
- PATENT ABSTRACTS OF JAPAN vol. 1995, no. 08, 29 September 1995 (1995-09-29) & JP 07 124505 A (CHUGAI PHARMACEUT CO LTD), 16 May 1995 (1995-05-16)
- PATENT ABSTRACTS OF JAPAN vol. 017, no. 113 (C-1033), 9 March 1993 (1993-03-09) & JP 04 298262 A (KOJI TODA), 22 October 1992 (1992-10-22)

## Description

### Field of the Invention

The present invention relates generally to a method for repelling or eliminating harmful organisms by efficiently and economically emitting in the air an effective ingredient having functions of eliminating insects, eliminating mites, retarding insects' growth and repelling insects.

### Discussion of the Related Art

As to the method for repelling or eliminating harmful organisms by emitting an effective ingredient in the air, there have conventionally been known a method using a liquid heating transpiration apparatus; a method utilizing a so-called aerosol method
comprising atomising a chemical liquid by discharging a high-pressure gas together with the chemical liquid; and a method utilizing a so-called piezoelectric method comprising atomising a chemical liquid through ultrasonic waves requiring high output, and the like.

US 4,173,651 relates to method for killing insects and/or fungi comprising vaporizing in air an insecticidal and/or fungicidal agent with an electro-mechanical ultrasonic nebuliser.

Among these methods, recently, the method using a liquid heating transpiration apparatus, in particular, has been well used because of its convenience in not requiring replenishment of liquid for a long period of time. In this method, since the chemical liquid containing the effective ingredient is discharged by continuously heating the liquid, once the heating temperature is stabilized, the chemical liquid particles can be continuously fed in the air. The size of the chemical liquid particles discharged from the heating transpiration apparatus described above are extremely small, and the particles relatively quickly become floating and diffusing in the heat-ascending air flow. Particularly, a tightly closed room allows the chemical liquid particles to stay floating for a longer period of time. Together with the aforesaid continuous feeding of the chemical liquid particles, this method provides an advantage of a long-lasting effect for repelling or eliminating the harmful organisms.

On the other hand, there are the following disadvantages. Since the chemical liquid is discharged through indirect continuous heating thereof, a great amount of time must be taken between the start of use and the exhibition of the effects of the chemical liquid. Furthermore, since the method involves continuous heat generation, requiring high energy, it is practically impossible to use a battery or the like to drive the transpiration apparatus for a long period of time. Further, since the heated portion of the wick tends to be clogged by such causation as deterioration of the effective ingredient with heating, the transpiration amount of the chemical liquid decreases in the latter half of the service period. In view of the above, in order to achieve power saving, for instance, if the transpiration apparatus is intermittently driven, a long time is required until the effect of the chemical liquid is exhibited through a transpiration portion heated to a predetermined temperature, and hence, a desired effect or a desired power saving cannot be accomplished.

By contrast, the method for discharging the chemical liquid by the aerosol method does not require electrical energy for atomisation energy of the chemical liquid, and allows instantaneous discharge of the chemical liquid in large amounts. Unfortunately, however, the presently commercially available aerosol products require the manual push-button operation, and besides, a given amount of chemical liquid cannot be discharged without the use of a special constant flow valve. Furthermore, many of the aerosol products are designed for principally treating the spraying particles directly upon target pests. In consideration of transcutaneous penetration of the effective ingredient into insect bodies, the aerosol products generally has a large particle size. Therefore, there are the following disadvantages when the particle size is large: 1) Slow diffusion rate in the air; 2) fast falling velocities of the particles; and 3) polluting the region where the chemical liquid is applied.

One object of the present invention is to provide a method for repelling or eliminating a harmful organism capable of reducing energy required for releasing the chemical liquid for repelling or eliminating a harmful organism, having a high safety, and being stable for a long period of time.

These and other objects of the present invention will be apparent from the following description.

### SUMMARY OF THE INVENTION

The present invention provides a method for repelling and eliminating a harmful organism according to claim 1.

### BRIEF DESCRIPTION OF THE DRAWINGS

The present invention will become more fully understood from the detailed description given hereinbelow and the accompanying drawing which are given by way of illustration only, and thus, are not limitative of the present invention, and wherein:

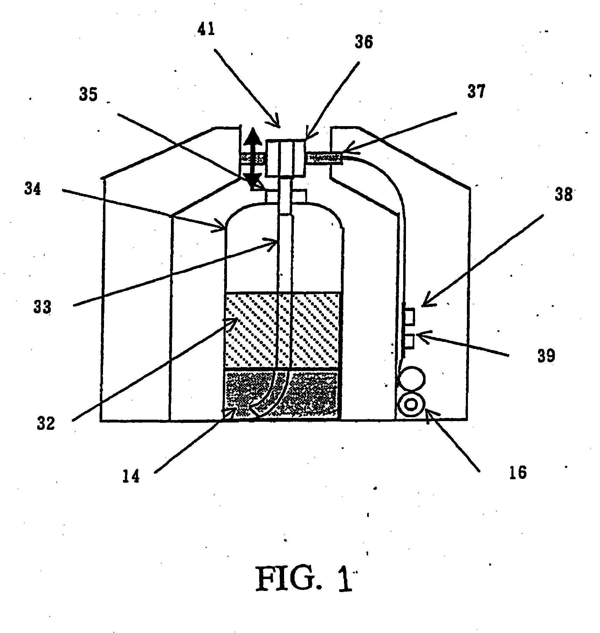

Figure 1 is a schematic view illustrating a chemical-liquid atomiser apparatus for the aerosol method including a control mechanism of open-close of the atomising head, and a control mechanism of spraying time interval and amount of sprayed liquid; and
   in the figure, 14 denotes a chemical liquid, 16 a battery, 32 a propellant, 33 a dip tube, 34 a pressure vessel, 35 an atomising head, 36 a button, 37 an electromagnetic valve, 38 an electromagnetic-valve control circuit, 39 a timer circuit, and 41 an emitting port.

### DETAILED DESCRIPTION OF THE INVENTION

In the present invention, the chemical liquid is emitted in the air as fine liquid droplets (fine , chemical liquid particles) by any one of various atomising means, so that the harmful organism may be repelled or eliminated by exhibition of an effect of effective ingredients in the chemical liquid.

As to the diameter of the fine chemical liquid particles, 90% by cumulative volume or more of the chemical liquid fine particles have a diameter of 20 µm or less, more preferably 90% by cumulative volume or more of the chemical liquid fine particles have a diameter of 15 µm or less, still more preferably 90% by cumulative volume or more of the chemical liquid fine particles have a diameter of 10 µm or less.

In the present specification, the diameter of the fine chemical liquid particles herein is a value determined by a particle size distribution analyser by means of a laser beam scattering at 25°C.

In this case, a suitable position for spraying the chemical liquid is set for measuring the diameter of the fine chemical liquid particles by taking into account a spraying output and the like such that the particle size distribution analyser may be allowed to provide favorable measurements. A suitable spraying position is spaced 10 to 50 cm away from the laser beam. It is suitable to measure the particle sizes immediately after the spraying, i.e. within three seconds from the spraying of the chemical liquid.

However, in a case where the use of the particle size distribution analyser cannot provide favorable measurements, such measurement values may be replaced by those given by a classifying device utilizing an Andersen sampler or the like.

In order to intermittently spray fine chemical liquid particles of diameters within a given range, for instance,the following spraying method may be employed.

For instance, a spraying method which utilizes a chemical-liquid atomiser apparatus using the aerosol method, the apparatus comprising a pressure vessel including an openable spray opening, and a chemical liquid sealed in the pressure vessel together with a propellant can be employed. Preferable method of spraying the chemical liquid in the air is a method which utilizes a chemical-liquid atomiser apparatus further including a power source, an open/close control mechanism for the spray opening and a control mechanism of spraying time interval and amount of sprayed liquid, including an electromagnetic valve, a constant rate valve, and the like. In this case, the aforesaid pressure vessel seals in the chemical liquid together with the propellant, the chemical liquid consisting essentially of the effective ingredients or comprising a mixture of effective ingredients and a solvent. The size of the fine chemical liquid particles can be adjusted by mainly adjusting a ratio of an initial volume of the chemical liquid to the overall volume of the pressure vessel.

The chemical liquid usable in the present invention is not particularly limited as long as the chemical liquid contains an effective ingredient exhibiting such effects as to kill insects or mites, retard insects' growth, repel insects, and the like. Alternatively, the effective ingredient itself may be used as the chemical liquid. Examples of effective ingredient usable herein include pyrethroid insecticides, pyrethroid-like insecticides, organic phosphorus insecticides, carbamate insecticides, chloronicotine insecticides, insect growth retardants, and microbicides. In the present invention, these effective ingredients may be used alone or in admixture thereof. In many of these effective ingredients, there are present geometric isomers ascribed to the carboxylic acid component and optical isomers ascribed to asymmetric carbon atom in the carboxylic acid component or the alcohol component. In the present invention, the chemical liquid can contain any of these isomers and an admixture thereof. Concrete examples of the effective ingredient are listed as below.

Examples of the pyrethroid insecticides include allethrin, dl • d-T80-allethrin, dl • d-T-allethrin, d• d-T-allethrin, d•d-T80-prallethrin, phthalthrin, d-T80-phthalthrin, resmethrin, d-T80-resmethrin, d-T80-furamethrin, permethrin, phenothrin, fenvalerate, cypermethrin, d-T80-cyphenothrin, empenthrin, terallethrin, imiprothrin and the like.

Examples of the pyrethroid-like insecticides include Etofenprox and the like.

Examples of the organic phosphorus insecticides include Diazinon, fenitrothion, pyridafenthion, malathion, Dipterex, chlorpyrifos, fenthion, dichlorvos, propetanphos, Abate, prothiophos, phoxim and the like.

Examples of the carbamate insecticides include propoxur and the like.

Examples of the chloronicotine insecticides include imidacloprid, acetamiprorid and the like.

Examples of the insect growth retardants (IGR) include pyriproxyfen, cyromazine and the like.

Examples of the microbicides include sulfur (S), Daconil, MDC, Topsin-M, Sumilex and the like.

Miticides, repellents and other insectifuges may be employed depending upon its purposes.

Examples of the miticide include kelthane and the like.

Examples of the repellent include N, N-diethyl-m-toluamide, dimethyl phthalate, dibutyl 10 phthalate, 2-ethyl-1, 3-hexanediol, p-dichlorobenzene, and the like.

These effective ingredients may be used alone or in admixtures of two or more kinds.

The chemical liquid can be prepared by dissolving or mixing the above effective ingredient in a solvent.

Examples of the solvent usable in the present invention include aliphatic hydrocarbons, alicyclic hydrocarbons, aromatic hydrocarbons, halogenated hydrocarbons, alcohols, esters, ethers, and ketones, with a particular preference given to aliphatic saturated hydrocarbons having 5 to 18 carbon atoms. Incidentally, although the aliphatic unsaturated hydrocarbons are not desirable as a main component of the solvent owing to its unpleasant odor, there is no problem in adding the aliphatic unsaturated hydrocarbons to the aliphatic saturated hydrocarbon in an amount so as not to cause an unpleasant odor.

In addition, in the aliphatic saturated hydrocarbons, there is a tendency that the larger the number of carbon atoms, the more viscous the hydrocarbons, and in some cases, having a gel-like state or solid state at an ambient temperature. In such cases, since mal-effects are likely to be obtained when supplied or sprayed as a chemical liquid, the aliphatic saturated hydrocarbon used as a solvent preferably has 18 or less carbon atoms. From the viewpoint of flash point at an ambient temperature, the aliphatic saturated hydrocarbon used as a solvent preferably has 5 or more carbon atoms. In addition, as to the aliphatic unsaturated hydrocarbons, those having 10 to 14 carbon atoms are more preferable, and particularly those having 11 to 13 carbon atoms are more preferable.

Examples of commercially available solvent comprising these aliphatic saturated hydrocarbons as a main component include "No. 0 Solvent M" (manufactured by NIPPON OIL CO., LTD.), "No. 0 Solvent L" (manufactured by NIPPON OIL CO., LTD.), "No. 0 Solvent H" (manufactured by NIPPON OIL CO., LTD.), "NORMAL PARAFFIN YH-NP" (manufactured by NIKKO PETROCHEMICALS CO., LTD.), "NORMAL PARAFFIN SH-NP" (manufactured by NIKKO PETROCHEMICALS CO., LTD.), "DEOTOMIZOL A-1" (manufactured by Yoshitomi Pharmaceutical Industries, Ltd.), "IP SOLVENT 2028" (manufactured by IDEMITSU PETROCHEMICAL CO., LTD.), "IP SOLVENT 1620" (manufactured by IDEMITSU PETROCHEMICAL CO., LTD.), "IP SOLVENT 1016" (manufactured by IDEMITSU PETROCHEMICAL CO., LTD.), and "NEO-CHIOZOL" (manufactured by Sanko Kagaku Kogyo K.K.).

Moreover, it is extremely effective to use fats and oil produced mainly extracted from plants and animals and utilize as food additives and starting cosmetic materials from the viewpoint of toxicity. Particularly, those oils which are less likely to be solidified by oxidation, including semi-dry oils such as sesame oil, rapeseed oil, and soybean oil; and those oils which are not solidified including non-dry oils such as camellia oil and olive oil are preferable.

In addition, a composition prepared by mixing one or more compounds selected from the group consisting of alcohols, aldehydes, ketone, and carboxylic acids as a solvent with water together with an effective ingredient may be used as a chemical liquid, or alternatively, a composition prepared by emulsifying and suspending in water the effective ingredient together with a surfactant may be used a chemical liquid. Since the above composition contains water, there is less danger against fire.

Incidentally, there may be other components besides the effective ingredients and solvents included in the chemical liquid. For example, in addition to the above effective ingredients, other components include stabilizing agents such as BHT and 2,2'-methylenebis(4-ethyl-6-t-butylphenol),: synergists such as 6-propyl-piperonyl ether, octachlorodipropyl ether, isobornyl thiocyan acetate, N-octylbicycloheptene carboxiimide, and N-(2-ethylhexyl)-1-isopropyl-4-methylenebicyclo(2,2,2)oct-5-en-2,3-carboxiimide; animal-derived or plant-derived natural perfumes; artificial perfumes made of hydrocarbons, alcohols, phenols, aldehydes, ketones, lactones, oxides, esters, and like; bactericidal-antifungal agents such as 0-phenylphenol, isopropylmethylphenol, 2-chloro-4-phenylphenol, and thymol.

The amount of sprayed effective ingredient as contained in the chemical liquid is not particularly limited the present invention. The amount of effective ingredient is suitably specified depending upon a target harmful organism, a type of effective ingredient used and an expected effect. The target harmful organisms include flies, mosquitoes, cockroaches, fleas, mites, moth flies, centipedes, ants, aphids, spider mites, and the like. The target harmful organisms include fungi such as *Erysiphales, Melanconiaceae, Sclerotiniaceae, Monhliaceae, Tuberculariaceae* and the like.

The preferred amount of sprayed effective ingredient in the chemical liquid ranges from 0.01 to 20 mg/h per 30 m³. The amount of sprayed-effective ingredient more preferably ranges from 0.05 to 20 mg/h per 30 m³, and particularly preferably ranges from 0.1 to 15 mg/h per 30 m³.

By way of an example, where d•d-T80-prallethrin is used as an effective ingredient for preventing mosquitoes from biting or eliminating them, the effective ingredient is preferably used in amounts in a range from 0.04 to 3.0 mg/h per 30 m³, and more preferably from 0.1 to 1.0 mg/h per 30 m³. Where another effective ingredient is used for the same purpose, the amount thereof is suitably selected from a range from 0.01 to 20 mg/h per 30 m³ depending upon a degree of activity of the effective ingredient used.

In order to set a desired amount of the effective ingredient to be sprayed, a spraying time, a spraying time interval, a size of fine chemical liquid particles and an amount of the chemical liquid emitted by one spray may be adjusted.

In the present invention, the chemical liquid is intermittently sprayed in the air and a stable effect to repel or eliminate harmful organisms is ensured over an extended period of time.

As described in the foregoing, in the present invention, by setting the particle size such that those having a particle size of 20 µm or less, based on volume cumulative distribution (volume cumulative percent), account for 90% or more of the entire particles, the falling velocity of the fine particles and the amount of fallen particles are decreased, thereby allowing the particles to be diffused in a wider area and also ensuring that the effective ingredient stays while floating in the air for a longer time period.

Additionally, the sprayed fine chemical liquid particles exhibit a behavior to gradually decrease in size because a main solvent evaporates from individual particles floating in the air. This leads to an extended floating time of the particles, i.e. an extended duration of effective period and an increased diffusibility of the particles in the air. The tendency of the particles decreasing in size varies depending upon the types of solvent and propellant used and therefore, a suitable solvent and propellant can be selected as desired.

If a group of particles greater than 20 µm in size is present in a volume cumulative percent of more than 10%, an amount of particles quick to fall increases and besides, such particles fall before exhibiting the aforesaid behavior to decrease in size while staying afloat in the air. This results in a lowered concentration of the effective ingredient in air (concentration in air) as well as in a decreased floatability or diffusibility of thereof and hence, a desired effect cannot be attained. In addition, there is increased pollution of a region where the chemical liquid is applied.

In a space where the harmful organism in the present invention is repelled or eliminated, the space including a house (residence), bungalow, tent, shop, greenhouse, vinyl house, warehouse and, the like, there is much air flow so that a favorable diffusibility of the effective ingredient is ensured by producing chemical liquid particles of a size specified by the present invention. Furthermore, by the aforesaid effect to extend the floating time of the particles, the continuous atomisation of the chemical liquid is not necessitated. Thus, energy consumption is reduced through intermittent atomisation of the chemical liquid, and moreover, safety is enhanced. The method for repelling or eliminating harmful organism of the present invention is quite advantageous in that an effect as indicated by the effective ingredient is safely and efficiently exhibited and maintained over an extended period of time in a power-saving manner. Therefore, the atomiser apparatus used in the method of the present invention can operate on a battery, such as dry batteries, so that the portability thereof is remarkably facilitated. As a result, the freedom of application of the present invention is dramatically increased.

The method of the present invention adapted for intermittent atomisation gives improvements to the problems of the conventional methods: the aerosol method suffering substantial chemical liquid loss due to greater particle sizes of the sprayed liquid obtained by the method; the liquid-heating transpiration method requiring high energy for continuous transpiration of the liquid even though the resultant particles are small in size; and the ultrasonic atomisation method requiring high energy for instantaneously atomising a large amount of liquid even though the resultant particles are small in size.

In the case of the intermittent atomisation, a time interval between a spray and the next spray (spraying interval) is not particularly limited. The time interval, for example, preferably ranges from 3 seconds to 60 minutes, more preferably from 10 seconds to 30 minutes, and particularly preferably from 20 seconds to 15 minutes. From the viewpoint of energy saving, the time interval of more than 3 seconds is preferred. From the viewpoints of safety and sustainability of the floating chemical liquid particles, the time interval of less than 60 minutes is preferred.

A spraying time for each spray is not particularly limited. However, in consideration of power saving when the battery or the like is used as a power source, the spraying time, for instance, preferably ranges from 0.05 to 300 seconds, more preferably from 0.1 to 180 seconds, and particularly preferably from 0.2 to 60 seconds.

A chemical-liquid atomiser apparatus usable for the method of the present invention is preferably capable of instantaneously emitting a predetermined amount of the chemical liquid. Examples of a preferred atomiser apparatus include the aerosol atomiser apparatus as mentioned above.

A concrete description will hereinbelow be made on the atomising methods.

### Aerosol Method

In this method, the size of fine chemical liquid particles can be designed to have a predetermined range mainly by adjusting the volume ratio of a chemical liquid to the overall volume of a pressure vessel, the chemical liquid consisting essentially of the effective ingredient, the chemical liquid comprising the effective ingredient and a solvent. More specifically, an initial volume of the chemical liquid accounts for 15% or less of the overall volume of the pressure vessel, and more preferably 10% or less, and particularly preferably 5% or less. Limiting the initial volume ratio of the chemical liquid to 15% or less leaves an initial volume ratio of 85% or more for a propellant occupying the pressure vessel. This provides the fine chemical liquid particles further reduced in size. The propellant may be composed of a gas phase and a liquid phase or of a gas phase alone. In a case where the propellant is composed of the gas phase and liquid phase, it is preferred that a single homogeneous liquid phase is formed by uniformly dissolving or dispersing the chemical liquid in the liquid phase of the propellant. In a case where the propellant is composed of the gas phase alone, it is preferred that the effective ingredient is uniformly dissolved or dispersed in the chemical liquid.

In a case where the chemical liquid is atomised by this method, the chemical liquid may consist essentially of the effective ingredient, or it may comprise a mixture of the effective ingredient and a solvent. In a case where the chemical liquid comprises the above mixture, a concentration of the chemical liquid is adjusted so that the initial volume ratio of the chemical liquid to the volume of the pressure vessel is within the predetermined range.

A preferred propellant sealed within the pressure vessel include, for example, at least one selected from the group consisting of liquefied petroleum gas (LPG), dimethyl ether (DME) and halogenated hydrocarbons. However, the propellant is not particularly limited to the above and compressed carbon dioxide gas, compressed nitrogen gas, compressed air or the like may be used as long as the desired fine chemical liquid particles can be formed.

The pressure vessel with the openable atomising head usable in the present invention is not particularly limited. Similar vessels to those used for the conventional aerosol products can be used.

As a control mechanism of open-close of the atomising head and the control mechanism of spraying interval and amount of the sprayed liquid, there may be included, for example, a method for electrically controlling both the amount of sprayed liquid and the spraying interval by using an electromagnetic valve adapted to open the atomising head for a given time period, and a method for electrically controlling only the spraying interval, while controlling the amount of sprayed liquid by utilizing a constant rate valve.

By using such a chemical-liquid atomiser apparatus for the aerosol method, the chemical liquid can be automatically and intermittently sprayed.

Figure 1 is a schematic view illustrating a chemical-liquid atomiser apparatus for the aerosol method including a power source, a control mechanism of open-close of the atomising head, and a control mechanism of spraying time interval and amount of sprayed liquid. In Figure 1, the apparatus comprises a chemical liquid 14, a propellant (liquid phase) 32, a dip tube 33, a pressure vessel 34, an openable atomising head (valve) 35, a button 36, an electromagnetic valve 37, an electromagnetic-valve control circuit 38, a timer circuit 39, and a battery 16 as a power source. The button 36, the electromagnetic valve 37 and the electromagnetic-valve control circuit 38 constitute the control mechanism of open-close of the atomising head 35. The timer circuit 39 serves as a control mechanism of spraying time interval and spraying time. Incidentally, the chemical liquid 14 is dissolved in the propellant 32.

A signal intermittently generated by the timer circuit 39 is applied to the electromagnetic-valve control circuit 38 for controlling an operation of the electromagnetic valve 37. Pressing the button 36 by operating the electromagnetic valve 37 opens the atomising head 35, whereby the chemical liquid 14 together with the propellant 32 flow through the dip tube 33 to be sprayed from an emitting port 41.

### EXAMPLES

The present invention will be described in further by means of the following working examples, comparative examples and test examples, without intending to limit the scope or spirit of the present invention thereto.

### Examples 1 to 33 and Comparative Examples 1 to 6

As the effective ingredients, there were used d•d-T80-prallethrin (Etoc), dl•d-T80-allethrin (Pynamin Forte, simply referred hereinafter as "Pynamin f"), terallethrin (Knockthrin), d-T80-phthalthrin (Neo-Pynamin Forte, simply referred hereinafter as "Neo-Pynamin f"), and d-T80-cyphenothrin (Gokilaht). For each of effective ingredients, the size of fine chemical liquid particles, the spraying time interval, the amount of each effective ingredient atomised per hour were adjusted as shown in Tables 1 and 2 to carried out the test. Incidentally, the tables indicate the particle sizes in a simplified manner as described below. More specifically, 90% of particles (X-90) based on the volume cumulative distribution (volume cumulative percent) had a size of N µm, sizes of the particles were simply referred to "N µm". The spraying time interval was defined as a period between the start of a first atomisation and the start of the subsequent atomisation. The spraying time was set to be shorter than the spraying time interval.

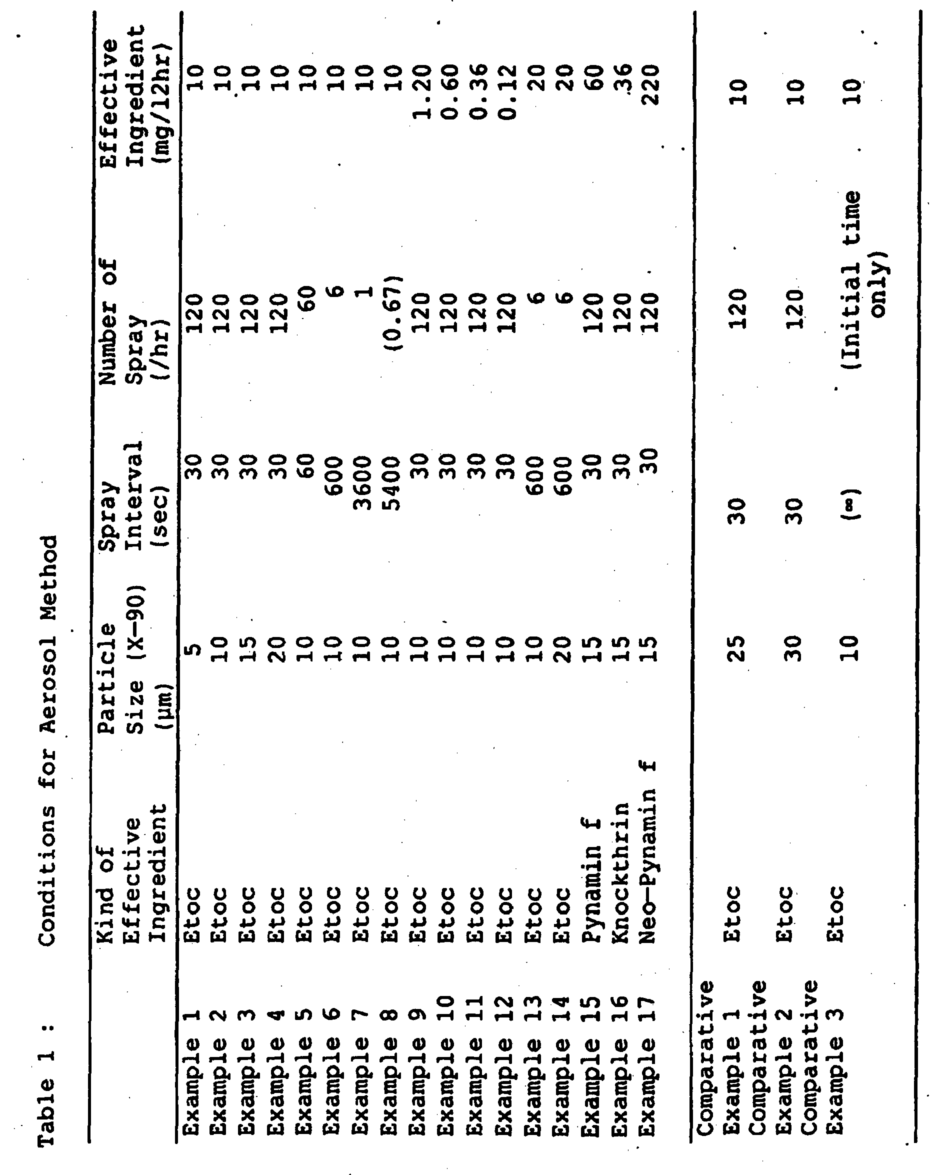

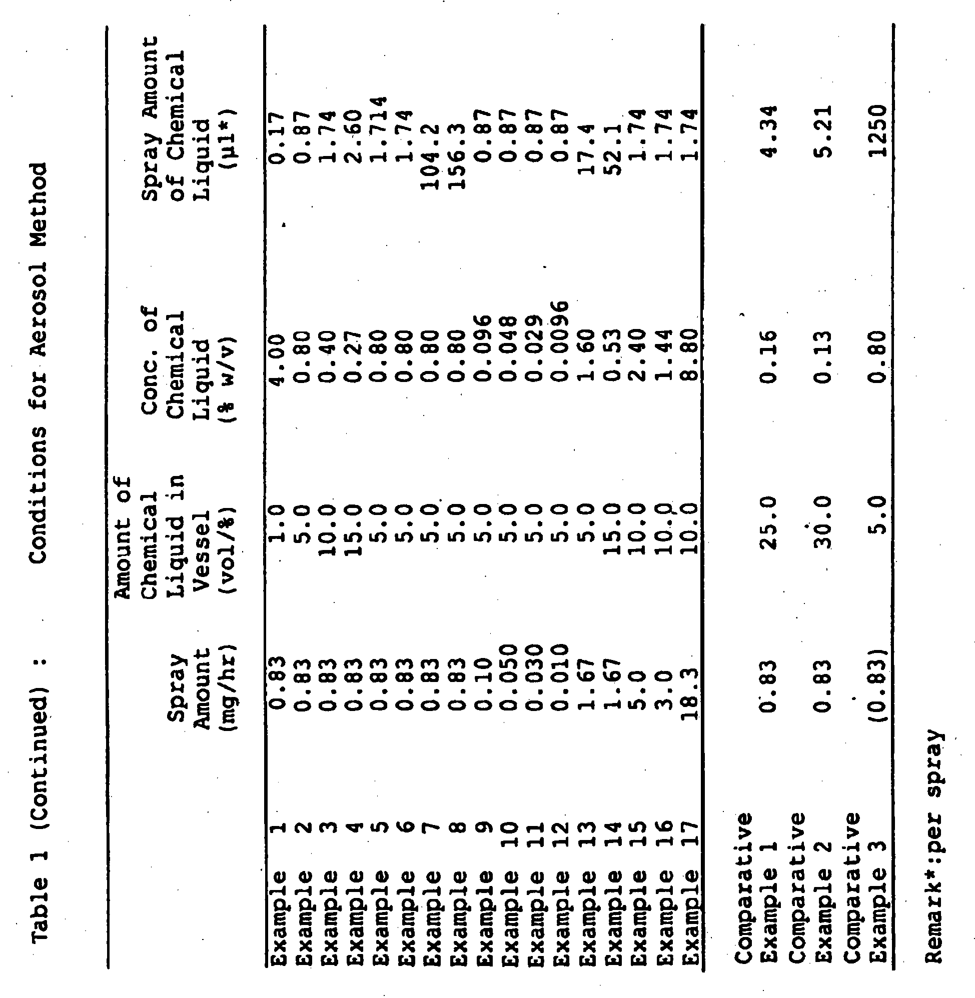

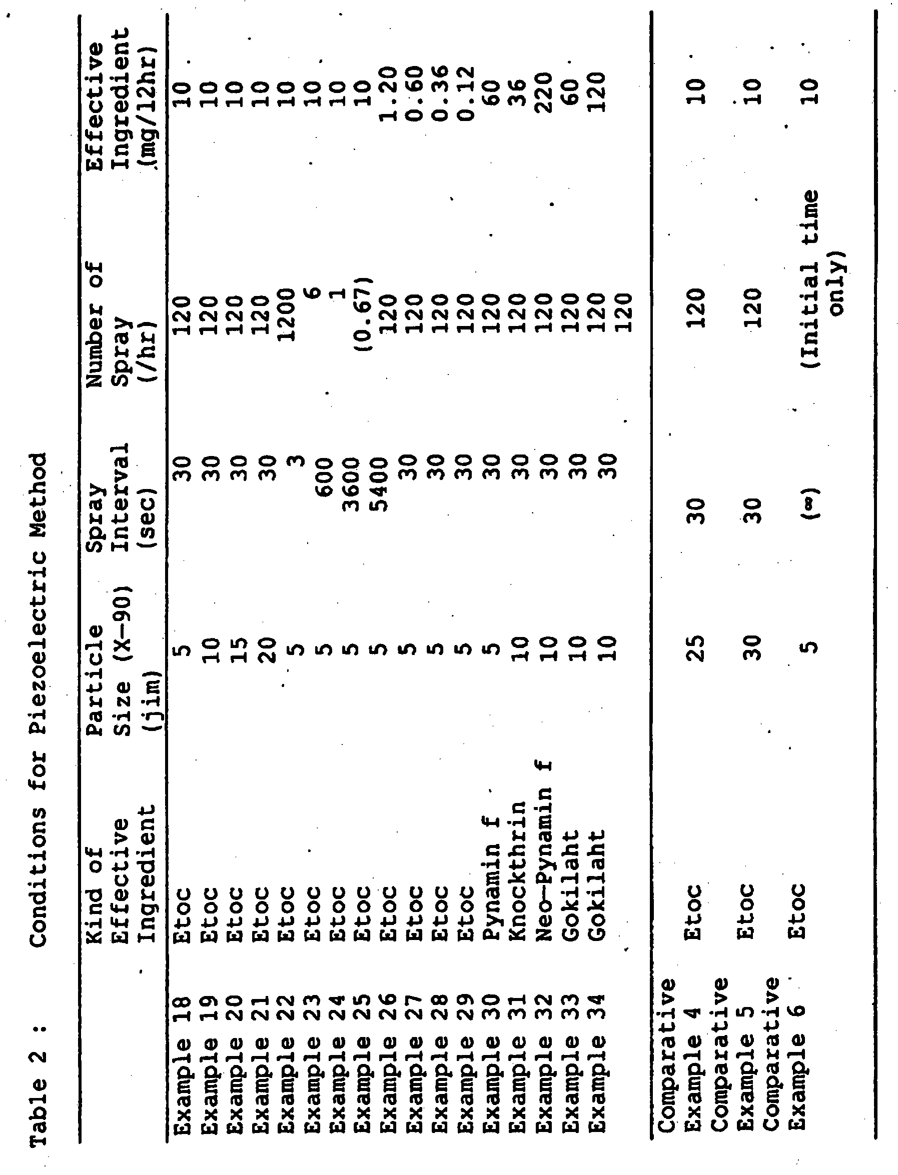

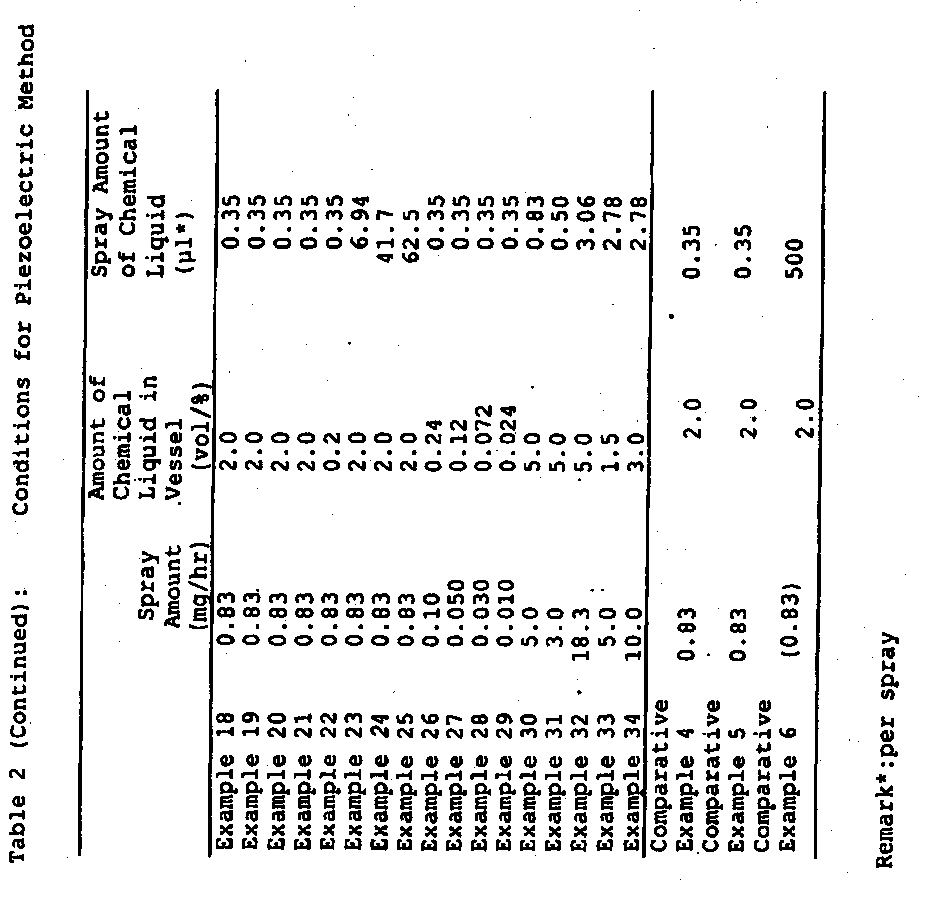

### Aerosol Method

An initial volume ratio of a chemical liquid was set to be within a range of 1.0 to 30% to a pressure vessel of 300 mL under the conditions given at Table 1. The chemical liquid was prepared by using ethanol as a solvent. In addition, DME was used as a propellant, and an internal pressure of the pressure vessel was adjusted to be from about 4 to about 5 kg/cm².

The diameter of the fine chemical liquid particles could be adjusted by a suitable type of valves and spray buttons to be used. In the present invention, it was adjusted by varying the volume ratio of the chemical liquid to the volume of the pressure vessel, the chemical liquid comprising the effective ingredient and a solvent. For example, when 90% of discharged chemical liquid particles based on the volume cumulative distribution had a size of about 5 µm, an initial volume ratio of the chemical liquid to the pressure vessel was set to be 1%. Similarly, when 90% of particles had a size of about 10 µm, an initial volume ratio of the chemical liquid was set to be 5%; when 90% of particles had a size of about 15 µm, an initial volume ratio of the chemical liquid was set to be 10%; when 90% of particles had a size of about 20 µm, an initial volume ratio of the chemical liquid was set to be 15%; when 90% of particles had a size of about 25 µm, an initial volume ratio of the chemical liquid was set to be 25%; and when 90% of particles had a size of about 30 µm, an initial volume ratio of the chemical liquid was set to be 30%.

As to the amount of discharge of the chemical liquid per one spray, the concentration of each of the effective ingredient in the chemical liquid was adjusted as follows.
For example,

| | |
|---|---|
| d•d-T80-prallethrin (Etoc) | 0.0096 to 4.0% (w/v) |
| dl•d-T80-allethrin (Pynamin f) | 2.4% (w/v) |
| terallethrin (Knockthrin) | 1.44% (w/v) |
| d-T80-phthalthrin (Neo-Pynamin f) | 8.8% (w/v) |

At the same time, a valve-opening time was controlled.

Incidentally, the apparatus shown in Figure 1 was used as an atomiser apparatus in this test.

### Test Example 1

This test was conducted under the setting conditions respectively specified for Examples 1 to 4 and 18 to 21 and Comparative Examples 1, 2, 4 and 5 as shown in Tables 1 and 2. The size of 90% of particles contained in the discharged chemical liquid particles based on the volume cumulative distribution was varied in a range from about 5 µm to about 30 µm to evaluate a knockdown effect for particles of respective sizes. As a sample insect, 100 female imagines of *Culex pipiens pallens* were released in a test room of about 8-tatami, i.e. about 30 m³, and the number knocked down insects was counted with the passage of time from release. From the results obtained, KT₅₀ values calculated according to Bliss' Probit Method were compared. The KT₅₀ value represented a time period required for knocking down 50% of the sample insects. Hence, the smaller the KT₅₀, the greater the knockdown effect, i.e. the effect for repelling or eliminating a target harmful organism.

Measurement points to count the knocked down insects were set to times after 30 minutes, one hour, 3.5 hours, 7 hours, and 12 hours from the start of the test. In the piezoelectric method, an additional measurement point was set to a time after 300 hours from the start, of the test. The evaluation of each case was made in the manner aforementioned.

A degree of pollution of the room floor with the chemical liquid was evaluated through a visual inspection after the operation under each condition. The inspection was performed after a lapse of 120 hours from the start of the test with respect to the aerosol method and after a lapse of 360 hours with respect to the piezoelectric method. The following evaluations were made for each rank:
- O:: Pollution of a degree that the floor was not changed in color or slightly changed;
- Δ:: Pollution of a degree that the floor was somewhat changed in color or became somewhat greasy; and
- x:: Pollution of a degree that the floor became greasy. The results are shown in Tables 3 and 4.

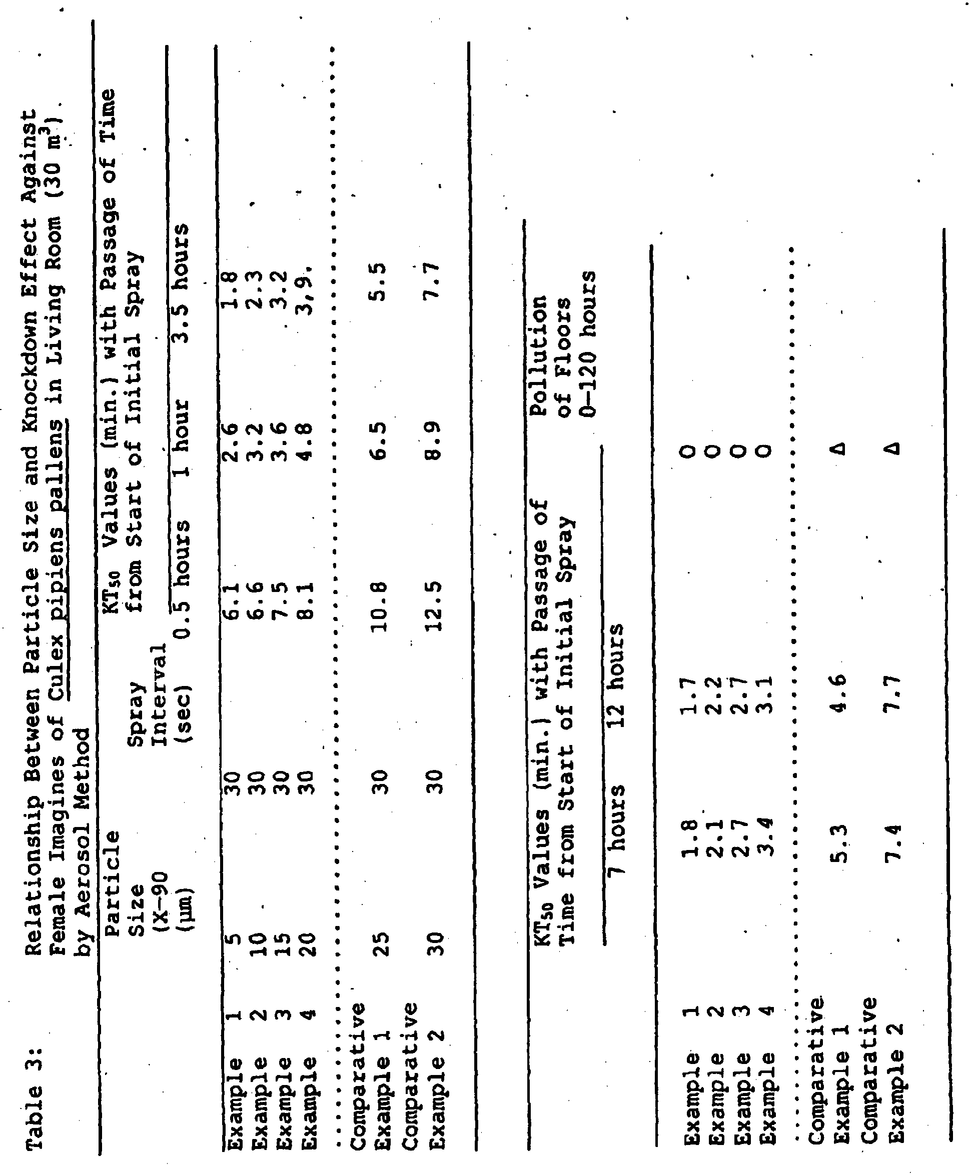

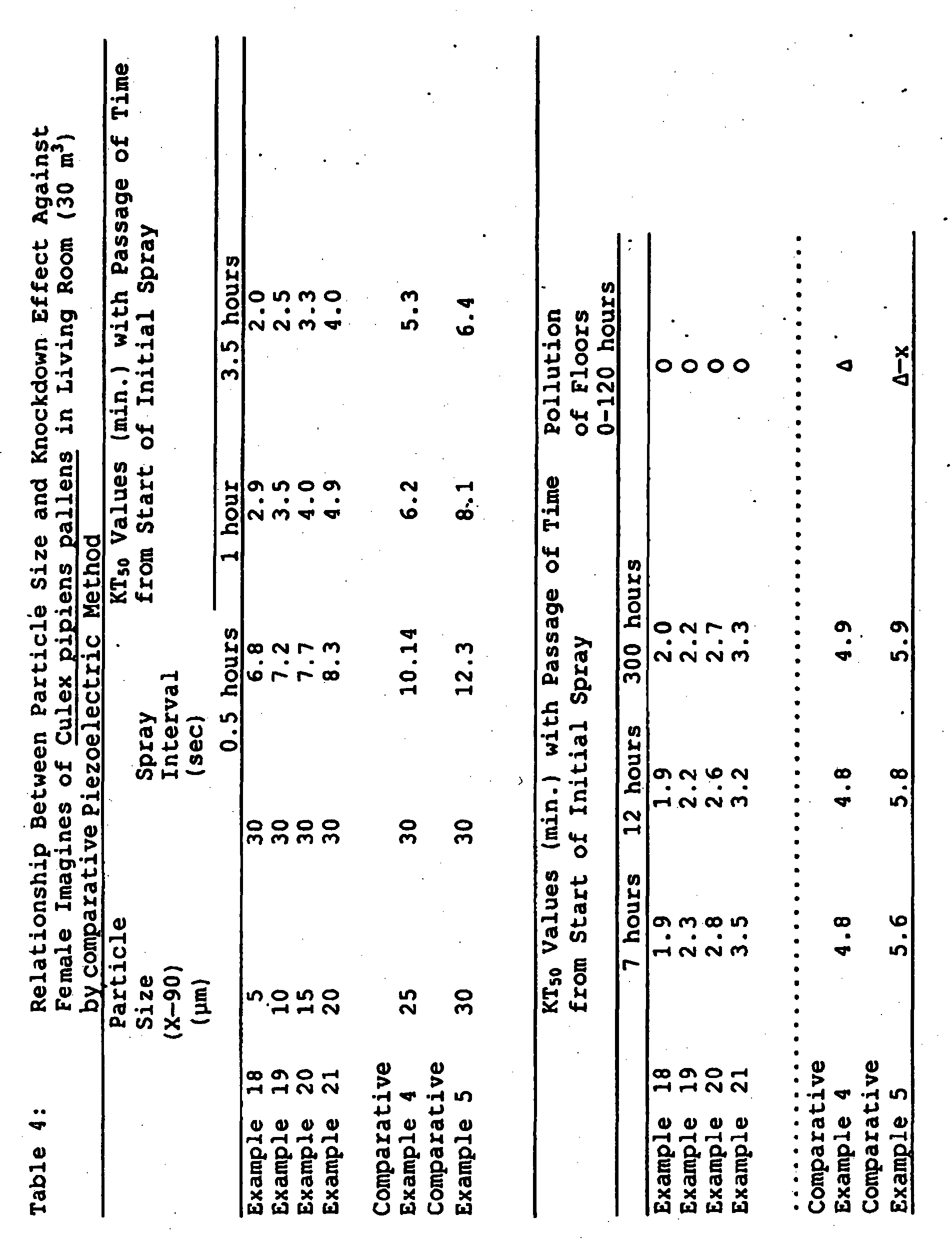

It is clear from the results that smaller the particle size, larger the knockdown effect and earlier the time in which the effect from the start of spraying was exhibited. Particularly when 90% of discharged chemical liquid particles based on the volume cumulative distribution have a size of about 20 µm or less, the most effective knockdown effect can be confirmed practically.

### Test Example 2

This test was conducted under the setting conditions respectively specified for Examples 5 to 8 and 22 to 25 and Comparative Examples 3 and 6 as shown in Tables 1 and 2. The size of 90% of discharged chemical liquid particles based on the volume cumulative distribution was about 10 µm for the aerosol method, and about 5 µm for the piezoelectric method to evaluate a knockdown effect for each spraying interval under conditions that spraying intervals was set between 3 and 5400 sec.

The method for evaluating the knockdown effect and the degree of pollution on the floor were carried out by method described in Test Example 1.

The results are shown in Tables 5 and 6.

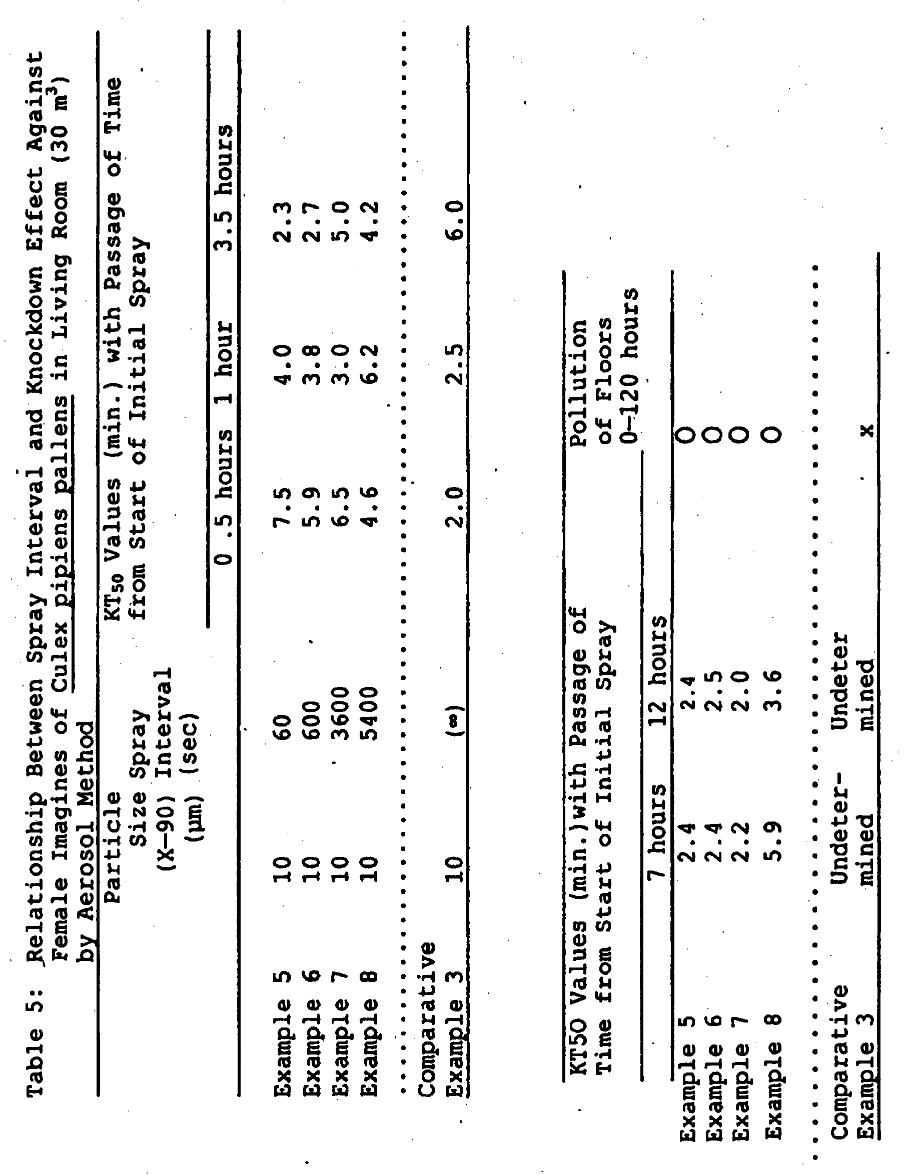

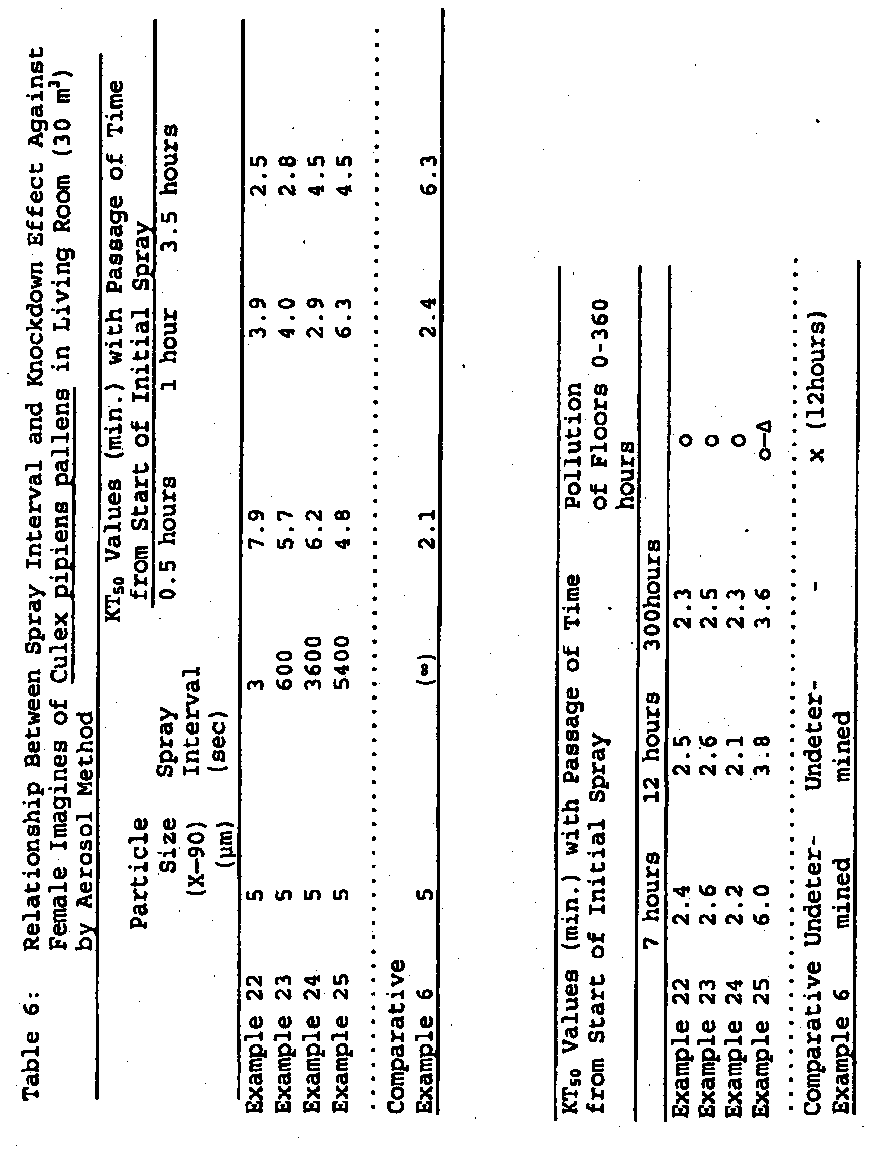

It is clear from the results that although a knockdown effect can be obtained when the spraying interval of the chemical liquid was set at 3600 seconds, namely 60 minutes, depending upon the duration of the passage of time, there are observed deviations among the knockdown effect in immediately after spraying and in subsequent spraying. For instance, when spraying interval is 5400 sec., deviations tend to become large, so that the spraying interval is preferably within 3600 sec.

In addition, it is clear from Comparative Examples 3 and 6 that although the total amount of the effective ingredient until spraying for 12 hours is at the same level as Examples, the effect cannot be sustained for a long period of time, thereby showing usefulness in an intermittent spraying in an appropriate interval.

### Test Example 3

This test was conducted under the setting conditions respectively specified for Examples 3, 15 to 17, 19, and 30 to 32 as shown in Tables 1 and 2 to evaluate a knockdown effect for different effective ingredients included in the chemical liquid particles released. The method for evaluating the knockdown effect was carried out by method described in Test Example 1.

The results are shown in Table 7.

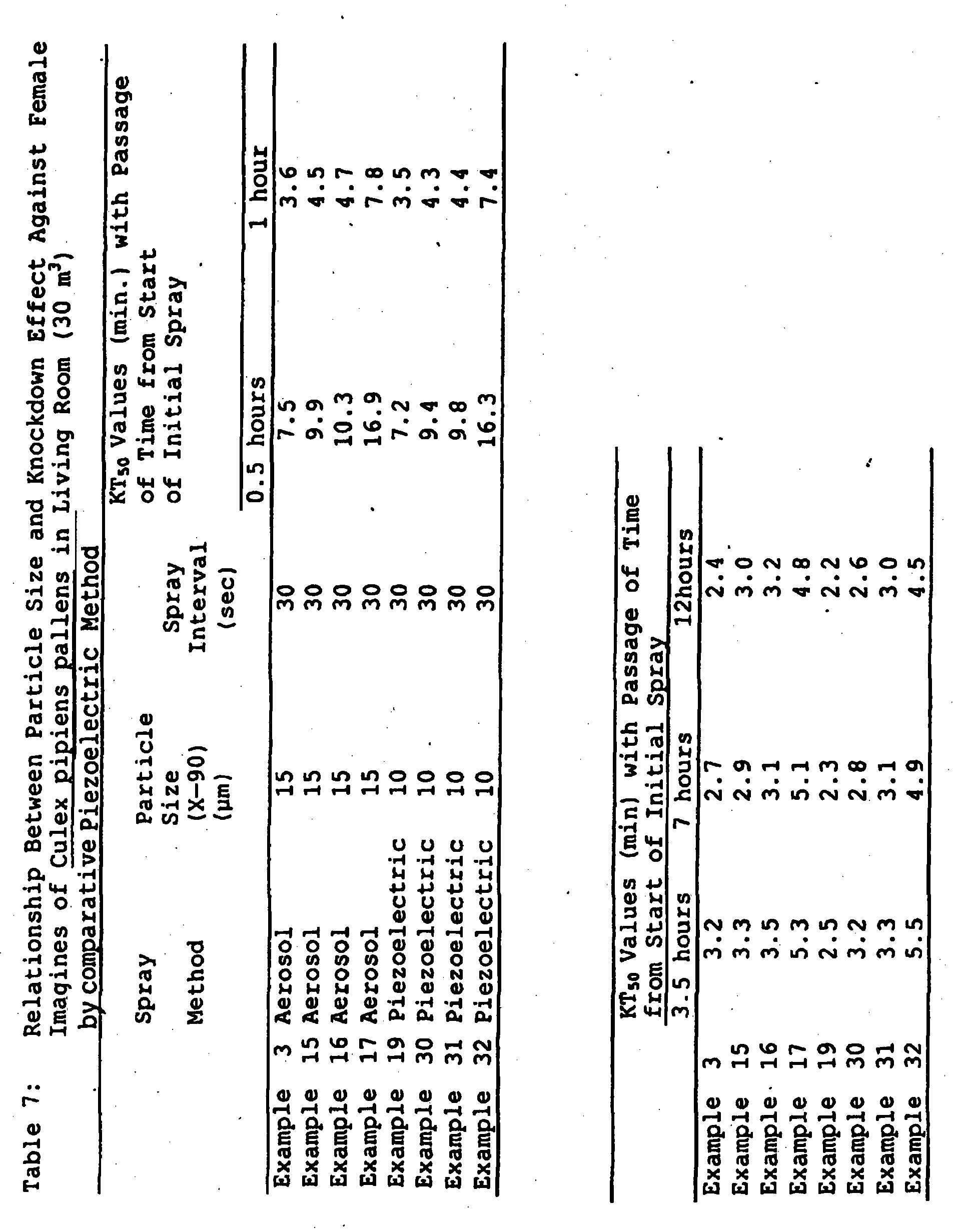

It is clear from the results that even if the effective ingredients were different, the effect activity owned by the effective ingredient itself can be exhibited, suggesting that an appropriate effect can be obtained by adjusting the amount of the effective ingredient sprayed depending upon the effect activity.

### Test Example 4

This test was conducted under the setting conditions respectively specified for Examples 13 and 14 as shown in Table 1 to evaluate a knockdown effect to female imagines of *Culex pipiens pallens* in a 16-tatami mat room (about 60 m³) when a spraying amount of the chemical liquid per spray was set for a size of 60 m³. The method for evaluating the knockdown effect was carried out by method described in Test Example 1.

The results are shown in Table 8.

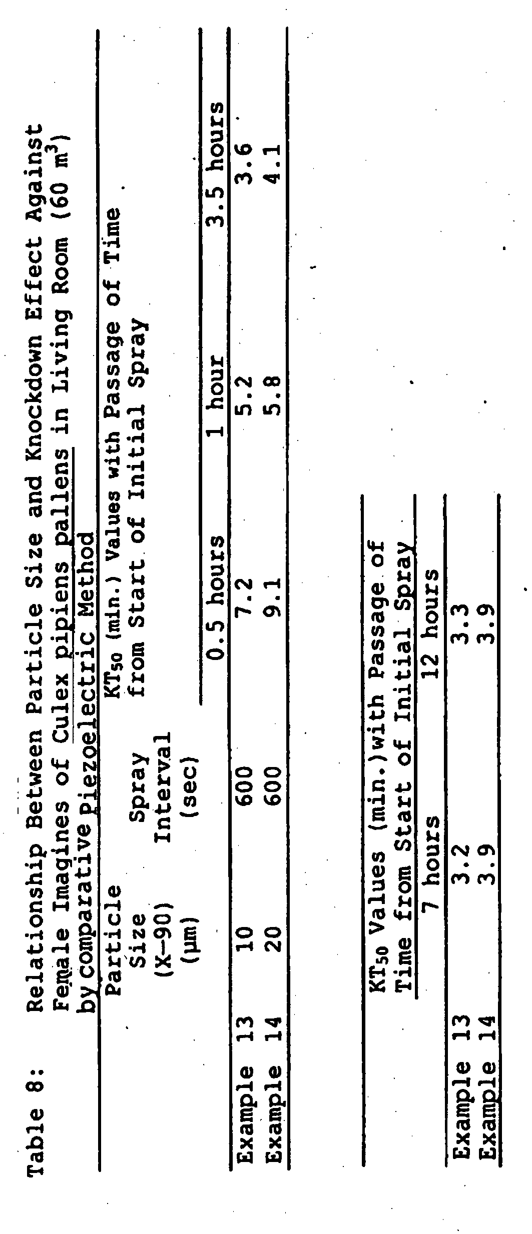

It is clear from the results that by doubling the spray amount of the chemical liquid of that used for 30 m³ room, desired effects can be obtained when doubling its volume.

### Test Example 5

This test was conducted under the setting conditions respectively specified for Examples 9 to 12 and 26 to 29 as shown in Tables 1 and 2, and studying on the relationship between the released amount of the effective ingredient per unit time and unit space and effect of inhibiting blood sucking of female imagines of *Aedes albopictus* after two hours from the start of test. The effect of inhibiting blood sucking was evaluated as follows. A living room-testing room was made ready by placing a rat fixed to a wire net as a source of blood sucking in the middle of a living area of 8-tatami mat room (about 30 m³), and the chemical liquid was intermittently sprayed over a period of 2 hours. Next, 100 individuals of female imagines of *Aedes albopictus* were released, and the total number of blood sucking after two hours from release was counted. In addition, as a control, a room without spraying the chemical liquid was prepared, and the number of blood sucking was counted.

The results are shown in Table 9.

**Table 9: Relationship Between Spray Amount and Effect of Inhibiting Blood Sucking Against Aedes albopictus in Living Room (30 m³) for Each Release**

| | | Spray Method | Amount of Chemical Released (mg/hr) | Percentage of Blood Sucking after 2 hours from Start of Initial Spraying (%) | | Percentage of Blood Sucking Inhibited (%) |
|---|---|---|---|---|---|---|
| | | | | Treated Zone | Untreated Zone | |
| Example | 9 | Aerosol | 0.1 | 0 | 53 | 100 |
| Example | 10 | Aerosol | 0.05 | 0 | 56 | 100 |
| Example | 11 | Aerosol | 0.03 | 6 | 55 | 89 |
| Example | 12 | Aerosol | 0.01 | 12 | 51 | 76 |
| Example | 26 | Piezoelectric | 0.1 | 0 | 50 | 100 |
| Example | 27 | Piezoelectric | 0.05 | 0 | 53 | 100 |
| Example | 28 | Piezoelectric | 0.03 | 7 | 58 | 88 |
| Example | 29 | Piezoelectric | 0.01 | 14 | 52 | 73 |

It is clear from the above test that in order to , obtain a minimal level of effect of inhibiting blood sucking for preventing the harm of blood sucking by mosquitos, the release amount of the effective ingredient per one hour per 30 m³ is desirably to be set at 0.01 mg or more.

### Test Example 6

This test was conducted under the setting conditions respectively specified for Examples 33 and 34 as shown in Table 2, in which the subject to be tested as harmful organisms was cockroaches. Incidentally, the test was carried out against *Periplaneta fuliginosa* and *Blattella germanica* in a 8-tatami mat room (equivalent to a space of about 30 m³). At four corners of the room were placed polyethylene cups containing subject insects to which vaseline was applied in the inner surface of the cup to avoid escape, and a box-shaped cover having dimensions of 30 cm x 30 cm x 48 cm with a hole of 6 cm x 6 cm on its side was placed thereon. Measurement points to count the knocked down insects were set to times after 3 hours, 6 hours, and 12 hours from the start of the' test.

The results are shown in Table 10.

**Table 10: Relationship of Knockdown Effect to Cockroaches in Living Room (30m³) by comparative Piezoelectric Method**

| Tested Organism | Particle Size (X-90) (µm) | Spray Interval (sec) | Knockdown Percentage (%) with Passage of Time from Initial Spraying | | |
|---|---|---|---|---|---|
| | | | 3 hours | 6 hours | 12 hours |
| Example 33 Periplaneta fuliginosa | 10 | 30 | 48 | 95 | 100 |
| Example 34 Periplaneta fuliginosa | 10 | 30 | 63 | 100 | 100 |
| Example 33 Blattella germanica | 10 | 30 | 28 | 93 | 100 |
| Example 34 Blattella germanica | 10 | 30 | 40 | 100 | 100 |

It is clear from the above test that its effect is also confirmed against cockroaches, showing that similar usefulness to mosquitoes can be found for harmful organisms other than mosquitoes.

Since the piezoelectric chemical-liquid spraying apparatus which does not belong to the present invention has easy exchange operation of the chemical liquid, having substantially no liquid leakage upon chemical liquid exchange, and having excellent spraying stability. In addition, according to the present invention, since the chemical liquid can be intermittently sprayed, the remarkable reduction in energy consumption and in the amount of the chemical liquid used can be achieved. Moreover, since batteries, etc. can be used, the method of the present invention has a wide range of applicability.

## Claims

1. A method for repelling and eliminating a harmful organism comprising spraying in the air a chemical liquid **characterised in that** the atomised chemical liquid is sprayed intermittently by using an aerosol atomiser apparatus comprising a pressure vessel having an openable spray opening, and including a chemical liquid being sealed therein together with a propellant, wherein an initial volume of the chemical liquid accounts for 15% or less of the overall volume of the pressure vessel, said chemical liquid containing an effective ingredient as atomised chemical liquid fine particles, wherein the resulting atomized chemical liquid fine particles have a particle size distribution in which 90% by cumulative volume of the chemical liquid fine particles based on volume cumulative distribution has a particle size of 20µm or less.

2. The method according to claim 1, wherein a spraying time interval is from 3 seconds to 60 minutes.

3. The method according to claim 1 or 2, wherein an amount of sprayed liquid of an effective ingredient in the chemical liquid per hour is from 0.01 to 20 mg per a space of 30 m³.

4. The method according to any one of claims 1 to 3, wherein the effective ingredient is one or more compounds selected from the group consisting of pyrethroid insecticides, pyrethroid-like insecticides, organic phosphorus insecticides, carbamate insecticides, chloronicotine insecticides, insect growth retardants, and microbicides.

5. The method according to any one of claims 1 to 4, wherein the atomizer apparatus further comprises a power source, an open/close control mechanism for the spray opening and a control mechanism of spraying time interval and amount of sprayed liquid.

## Patentansprüche

1. Ein Verfahren zur Abwehr und Eliminierung eines Schadorganismus, umfassend das Sprühen einer chemischen Flüssigkeit in die Luft, **dadurch gekennzeichnet, dass** die versprühte chemische Flüssigkeit stoßweise unter Verwendung einer Aerosolzerstäubungsvorrichtung gesprüht wird, wobei die Vorrichtung einen Druckbehälter mit einer zu öffnenden Sprühöffnung aufweist und eine chemische Flüssigkeit darin verschlossen zusammen mit einem Treibgas enthält, worin das Anfangsvolumen der chemischen Flüssigkeit 15 % oder weniger des Gesamtvolumens des Druckbehälters beträgt, wobei die chemische Flüssigkeit einen aktiven Wirkstoff als feinteilige Partikel einer versprühten chemischen Flüssigkeit umfasst, wobei die resultierenden feinteiligen Partikel der versprühten chemischen Flüssigkeit eine Partikelgrößenverteilung aufweisen, in welcher 90 % des kumulativen Volumens der feinteiligen Partikel der chemischen Flüssigkeit bezogen auf die kumulative Volumenverteilung eine Partikelgröße von 20 µm oder weniger aufweisen.

2. Das Verfahren nach Anspruch 1, worin ein Sprühzeitintervall von 3 Sekunden bis 60 Minuten dauert.

3. Verfahren nach Anspruch 1 oder 2, worin eine Menge an gesprühter Flüssigkeit eines aktiven Wirkstoffs in der chemischen Flüssigkeit pro Stunde 0,01 bis 20 mg für eine Fläche von 30 m³ beträgt.

4. Verfahren nach einem der Ansprüche 1 bis 3, worin der aktive Wirkstoff eine oder mehrere Verbindungen sind, ausgewählt aus der Gruppe, bestehend aus Pyrethroid-Insektiziden, Pyrethroid-ähnlichen Insektiziden, Organophosphor-Insektiziden, Carbamat-Insektiziden, Chloronikotinyl-Insektiziden, Insektizidwachstumshemmer und Mikrobiziden.

5. Verfahren nach einem der Ansprüche 1 bis 4, worin die Zerstäubungsvorrichtung darüber hinaus eine Stromquelle, einen Öffnungs-/Schließ-Kontrollmechanismus für die Sprühöffnung und einen Kontrollmechanismus für das Sprühzeitintervall und die Menge an gesprühter Flüssigkeit umfasst.

## Revendications

1. Procédé de répulsion et d'élimination d'un organisme nuisible comprenant les étapes consistant à pulvériser dans l'air un liquide de produit chimique, **caractérisé en ce que** le liquide chimique atomisé est pulvérisé par intermittence en utilisant un appareil atomiseur d'aérosol comprenant un récipient sous pression ayant un orifice de pulvérisation ouvrable et comprenant un liquide de produit chimique hermétiquement contenu dans celui-ci, conjointement avec un propulseur, dans lequel un volume initial du liquide chimique représente 15% ou moins du volume total du récipient sous pression, ledit liquide de produit chimique contenant un principe actif sous forme de particules liquides fines atomisées de produit chimique, dans lequel les particules liquides fines atomisées de produit chimique résultantes présentent une distribution de taille de particules dans laquelle 90% en volume cumulé des particules liquides fines de produit chimique basées sur la distribution cumulative en volume, possèdent une taille de particules de 20 µm ou moins.

2. Procédé selon la revendication 1, dans lequel une durée de pulvérisation va de 3 secondes à 60 minutes.

3. Procédé selon la revendication 1 ou 2, dans lequel une quantité de liquide pulvérisé d'un principe actif dans le liquide chimique par heure va de 0,01 à 20 mg par volume de 30 m3.

4. Procédé selon l'une quelconque des revendications 1 à 3, dans lequel le principe actif est un ou plusieurs composés choisis dans le groupe constitué d'insecticides pyréthrinoïdes, d'insecticides analogues aux pyréthrinoïdes, d'insecticides phosphoreux organiques, d'insecticides carbamates, d'insecticides chloronicotiniques, de ralentisseurs du rythme de croissance des insectes et de microbicides.

5. Procédé selon l'une quelconque des revendications 1 à 4, dans lequel l'appareil atomiseur comprend en outre une source d'énergie, un mécanisme de commande ouverture/fermeture pour l'orifice de pulvérisation et un mécanisme de commande de durées de pulvérisation et de quantités de liquide pulvérisé.
